# EUROPEAN PATENT SPECIFICATION

(11) **EP 4 324 336 B1**
(45) Date of publication and mention of the grant of the patent: **01.04.2026**
(21) Application number: 22788048.1
(22) Date of filing: 30.03.2022
(51) Int. Cl.: A61K 31/4172, A61P 39/06, A61P 17/18, A61K 47/22, A23L 33/10, A61K 31/7028

(54) **L-ERGOTHIONEINE-CONTAINING COMPOSITION**
L-ERGOTHIONEINHALTIGE ZUSAMMENSETZUNG
COMPOSITION CONTENANT DE LA L-ERGOTHIONÉINE

(30) Priority: 14.04.2021 JP 2021068583
(43) Date of publication of application: 21.02.2024
(73) Proprietor: Nagase & Co., Ltd., Osaka-shi, Osaka 550-8668 (JP)
(72) Inventor: MATSUMOTO,Jun, Kobe-shi, Hyogo 651-2241 (JP); NAKATANI,Takeshi, Kobe-shi, Hyogo 651-2241 (JP)
(74) Representative: Cabinet Beau de Loménie
(86) International application number: PCT/JP2022/015935
(87) International publication number: WO 2022/220123

(56) References cited:
- EP-A1- 3 751 001
- WO-A1-2019/163767
- US-A- 5 438 151
- US-A1- 2015 225 755
- HALLIDAY, N.M. ; HARDIE, K.R. ; WILLIAMS, P. ; WINZER, K. ; BARRETT, D.A.: "Quantitative liquid chromatographytandem mass spectrometry profiling of activated methyl cycle metabolites involved in LuxS-dependent quorum sensing in Escherichia coli", ANALYTICAL BIOCHEMISTRY, vol. 403, no. 1-2, 1 August 2010 (2010-08-01), Amsterdam, NL , pages 20 - 29, XP027118749, ISSN: 0003-2697, DOI: 10.1016/j.ab.2010.04.021

## Description

### TECHNICAL FIELD

The present invention relates to an L-ergothioneine (EGT)-comprising composition and to its use. More specifically, the present invention relates to a composition as defined in claim 1.

### BACKGROUND ART

L-ergothioneine (herein, sometimes referred to as "EGT"), a kind of sulfur-containing amino acid, is known to have various physiological activities including an antioxidant capacity.

For example, Non-Patent Document 1 discloses that EGT has a function of improving the effect of immunotherapy by a cancer vaccine including a tumor-associated antigen (TAA) and an adjuvant. Patent Document 1 discloses that EGT has a function of treating bacterial infections. In Experiment 1 of Patent Document 1, it has been confirmed that EGT at 5 mM and 50 mM suppresses the growth of Escherichia coli. Therefore, it can be understood that the suppression of bacterial infections described in Patent Literature 1 is achieved by direct suppression of the growth of bacteria by EGT. Patent document 2 discloses a method for producing ergothioneine, comprising culturing a bacterium belonging to the family Enterobacteriaceae having ergothioneine-producing ability in a culture medium, and collecting ergothioneine from the culture medium. S-ribosylhomocysteine is not mentioned in this document.

Non-Patent Document 2 discloses that the production of cytokines (IL-6, IL-12p40, IL-1β, IL-10) by mouse bone marrow-derived macrophages under stimulation conditions with a toll-like receptor ligand is promoted by 10 mM EGT and not by 1 mM EGT, and that in the co-culture of F4/80 macrophages and OT-II CD4⁺ T cells, Th17 polarization of OT-II CD4⁺ T cells is promoted by 30 mM EGT. As described above, Non-Patent Document 2 discloses that EGT at a high concentration of 10 mM or more activates macrophages.

Non-Patent Document 3 is a review of the physiological activities of EGT. Non-Patent Document 3 discloses that EGT is absorbed and accumulated in the bodies of animals and plants.

### PRIOR ART DOCUMENTS

### PATENT DOCUMENT

Patent Document 1: WO 2019/089878 Patent Document 2: EP 3751001 A1

### NON-PATENT DOCUMENTS

Non-Patent Document 1: S. Yoshida et al., Front. Immunol. 10: 671 (2019)
Non-Patent Document 2: PLoS ONE 12 (1): e0169360
Non-Patent Document 3: Bulletin of the College of Agriculture, Tamagawa University 1: 17-41 (2016)

### SUMMARY OF THE INVENTION

### PROBLEMS TO BE SOLVED BY THE INVENTION

However, the formulation stability in compositions comprising EGT has not yet been thoroughly investigated. In particular, the influence of other coexisting components in compositions comprising EGT has not been reported in detail.

### MEANS FOR SOLVING THE PROBLEMS

As a result of intensive studies in view of the above problems, a novel problem has been found in that in a case of a composition comprising EGT in which S-ribosylhomocysteine (SRH) coexists, coloring of the composition comprising EGT occurs, which has not been reported so far.

In order to solve this novel problem, the present inventors have found that in a composition comprising EGT and SRH, reducing the content of the SRH can suppress coloring of the composition, thereby completing the present invention.

That is, the present invention provides the following composition.
[1] A composition comprising L-ergothioneine and S-ribosylhomocysteine, wherein the content of S-ribosylhomocysteine is 15 parts by mass or less with respect to 100 parts by mass of the L-ergothioneine.
[2] The composition according to f [1], in which the content of S-ribosylhomocysteine is 0.001 parts by mass or more with respect to 100 parts by mass of L-ergothioneine.
[3] A composition comprising L-ergothioneine and S-ribosylhomocysteine, in which the content of S-ribosylhomocysteine is 5 parts by mass or less with respect to 100 parts by mass of L-ergothioneine.
   The present invention also relates to the following method.
[7] A method for imparting a coloring-suppressing effect to a composition comprising L-ergothioneine and S-ribosylhomocysteine, wherein the method comprises setting the content of S-ribosylhomocysteine in the composition to 15 parts by mass or less with respect to 100 parts by mass of L-ergothioneine.

### EFFECT OF THE INVENTION

In compositions comprising EGT, S-ribosylhomocysteine (SRH) was found as an ingredient that affects the coloring issue of the composition. Accordingly, in the composition in which EGT and SRH coexist, coloring of the composition can be suppressed by reducing the content of SRH.

### BRIEF DESCRIPTION OF THE DRAWINGS

Fig. 1 is a photograph showing a degree of coloring of a sample stored for six months before being dissolved in water in Test Example 1.
Fig. 2 is a photograph showing a degree of coloring of a sample stored for six months after being dissolved in water in Test Example 1.

### MODE FOR CARRYING OUT THE INVENTION

The composition of the present invention is a composition comprising L-ergothioneine (EGT) and S-ribosylhomocysteine (SRH), wherein the content of S-ribosylhomocysteine is 15 parts by mass or less with respect to 100 parts by mass of the L-ergothioneine.

### [L-ergothioneine (EGT)]

EGT is a histidine derivative (N,N,N-trimethyl-L-2-thiohistidine), whose structure is represented by the following formula (1).

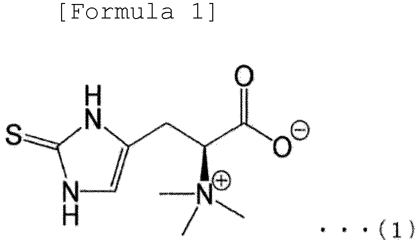

EGT can be obtained by a publicly known method such as a synthesis method, an extraction method, or a fermentation method, and also a commercially available product can be obtained and used.

Examples of the commercially available product of EGT include L-ergothioneine (manufactured by Tetrahedron).

### [S-Ribosylhomocysteine (SRH)]

The structure of SRH is expressed by the following Formula (2).

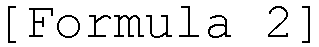

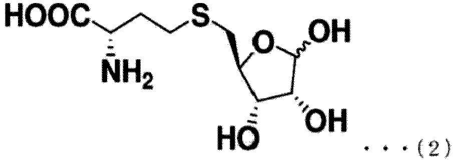

The SRH can be obtained by a publicly known method such as a synthesis method, an extraction method, or a fermentation method.

The EGT and/or SRH may be in the form of a free form or in the form of a salt. The salt of EGT and/or SRH may be a salt formed with a carboxyl group in these structures, or may be a salt formed with a trimethylamino group or an amino group. The EGT or SRH may be a solvate such as a hydrate.

The salt of EGT and/or SRH is not particularly limited as long as the salt is a pharmacologically or physiologically acceptable salt, and specific examples thereof include an organic acid salt, an inorganic acid salt, a salt with an organic base, and a salt with an inorganic base. Examples of the organic acid salt include monocarboxylates such as acetate, trifluoroacetate, butyrate, palmitate, and stearate; polyvalent carboxylates such as fumarate, maleate, succinate, and malonate; oxycarboxylates such as lactate, tartrate, and citrate; and organic sulfonates such as methanesulfonate, toluenesulfonate, and tosylate. Examples of the inorganic acid salt include hydrochloride, sulfate, nitrate, hydrobromide, and phosphate. Examples of the salt with an organic base include salts with organic amines such as methylamine, triethylamine, triethanolamine, diethanolamine, morpholine, piperazine, pyrrolidine, and ethylenediamine. Examples of the salt with an inorganic base include various salts such as salts with alkali metals such as sodium and potassium, alkaline earth metals such as calcium and magnesium, and metals such as aluminum. These salts of EGT and/or SRH may be used alone, or may be used in any combination of two or more thereof. The "pharmaceutically or physiologically acceptable salt" may include a solvate or hydrate of a salt.

In the following Examples, the present inventors have found a novel problem in that in a case of a composition comprising EGT in which SRH coexists, coloring of the composition comprising EGT occurs, which has not been reported so far. Furthermore, the present inventors have found that in a composition comprising EGT and SRH, reducing the content of the SRH can enhance the stability of the composition comprising EGT and suppress coloring of the composition. Aspects of the composition comprising EGT in which SRH coexists are not particularly limited. For example, SRH may be added to the composition comprising EGT, or may be endogenously comprised as, for example, impurities in an extraction method, or by-products in a synthesis method, a fermentation method, or the like.

Details of the mechanism of destabilization when SRH coexists in the composition comprising EGT are still unknown, but it is presumed that SRH is gradually decomposed to generate a decomposition product of the SRH, and the decomposition product may adversely affect the stabilization of the composition comprising EGT. Therefore, it is considered that reducing the content of SRH also leads to a reduction in a decomposition product of SRH, and consequently contributes to the stabilization of the composition comprising EGT. The decomposition product of SRH is not particularly limited, but examples thereof include an SRH dehydrated dimer.

In the composition of the present invention, "coloring is suppressed" means that the absorbance at 400 nm is reduced by reducing the content of SRH in the condition that the EGT content is constant, as compared with the composition comprising 20 parts by mass of EGT and SRH (hereinafter, also referred to as a reference composition.). The reduction in absorbance at 400 nm can be determined using a publicly known spectrophotometer. For example, the reduction in absorbance at 400 nm is preferably at least a 5% reduction, more preferably at least a 10% reduction, as compared with the reference composition.

In the composition of the present invention, as to the content ratio of SRH to EGT, the content of SRH is 15 parts by mass or less, preferably 10 parts by mass or less, more preferably 5 parts by mass or less, even more preferably 3 parts by mass or less, even more preferably 1 part by mass or less, even more preferably 0.5 parts by mass or less, even more preferably 0.1 parts by mass or less, particularly preferably 0.01 parts by mass or less, with respect to 100 parts by mass of EGT, from the viewpoint of suppressing coloring of the composition.

In addition, in the composition of the present invention, as to the content ratio of SRH to EGT, the content of SRH can be, for example, 0.0001 parts by mass or more, 0.001 parts by mass or more, 0.005 parts by mass or more, 0.01 parts by mass or more, or the like, with respect to 100 parts by mass of EGT, from the viewpoint of production efficiency of the composition, and the like.

In addition, in the composition of the present invention, as to the content ratio of SRH to EGT, the content of SRH can be, for example, 0.0001 to 15 parts by mass, 0.0001 to 10 parts by mass, 0.0001 to 5 parts by mass, 0.0001 to 1 parts by mass, 0.0001 to 0.5 parts by mass, 0.0001 to 0.1 parts by mass, 0.001 to 15 parts by mass, 0.001 to 10 parts by mass, 0.001 to 5 parts by mass, 0.001 to 1 parts by mass, 0.001 to 0.5 parts by mass, 0.001 to 0.1 parts by mass, 0.005 to 15 parts by mass, 0.005 to 10 parts by mass, 0.005 to 5 parts by mass, 0.005 to 1 parts by mass, 0.005 to 0.5 parts by mass, 0.005 to 0.1 parts by mass, 0.01 to 15 parts by mass, 0.01 to 10 parts by mass, 0.01 to 5 parts by mass, 0.01 to 1 parts by mass, 0.01 to 0.5 parts by mass, 0.01 to 0.1 parts by mass, and the like, with respect to 100 parts by mass of EGT, from the viewpoint of suppressing coloring of the composition, the viewpoint of stability of the composition, the viewpoint of production efficiency of the composition, and the like.

As described above, it is considered that reducing the content of SRH also leads to a reduction in a decomposition product of SRH, and consequently contributes to the stabilization of the composition comprising EGT. The decomposition product of SRH is not particularly limited, but for example, in a case of an SRH dehydrated dimer, as to the content ratio of SRH dehydrated dimer to EGT, the content of SRH dehydrated dimer is, for example, preferably 15 parts by mass or less, more preferably 10 parts by mass or less, still more preferably 5 parts by mass or less, even more preferably 3 parts by mass or less, even more preferably 1 part by mass or less, even more preferably 0.5 parts by mass or less, even more preferably 0.1 parts by mass or less, particularly preferably 0.01 parts by mass or less, with respect to 100 parts by mass of EGT, from the viewpoint of suppressing coloring of the composition.

In addition, in the composition of the present invention, as to the content ratio of SRH dehydrated dimer to EGT, the content of SRH dehydrated dimer can be, for example, 0.0001 parts by mass or more, 0.001 parts by mass or more, 0.005 parts by mass or more, 0.01 parts by mass or more, or the like, with respect to 100 parts by mass of EGT, from the viewpoint of production efficiency of the composition, and the like.

In addition, in the composition of the present invention, as to the content ratio of SRH dehydrated dimer to EGT, the content of SRH dehydrated dimer can be, for example, 0.0001 to 15 parts by mass, 0.0001 to 10 parts by mass, 0.0001 to 5 parts by mass, 0.0001 to 1 parts by mass, 0.0001 to 0.5 parts by mass, 0.0001 to 0.1 parts by mass, 0.001 to 15 parts by mass, 0.001 to 10 parts by mass, 0.001 to 5 parts by mass, 0.001 to 1 parts by mass, 0.001 to 0.5 parts by mass, 0.001 to 0.1 parts by mass, 0.005 to 15 parts by mass, 0.005 to 10 parts by mass, 0.005 to 5 parts by mass, 0.005 to 1 parts by mass, 0.005 to 0.5 parts by mass, 0.005 to 0.1 parts by mass, 0.01 to 15 parts by mass, 0.01 to 10 parts by mass, 0.01 to 5 parts by mass, 0.01 to 1 parts by mass, 0.01 to 0.5 parts by mass, 0.01 to 0.1 parts by mass, and the like, with respect to 100 parts by mass of EGT, from the viewpoint of suppressing coloring of the composition, the viewpoint of stability of the composition, the viewpoint of production efficiency of the composition, and the like.

In addition, in the composition of the present invention, the content of EGT is appropriately adjusted depending on the form and use of the formulation, the type and content of other components, and the like, and is not limited, but can be, for example, 0.000001 mass% or more with respect to the total amount of the composition, including 0.000005 mass% or more, 0.00001 mass% or more, 0.00005 mass% or more, 0.0001 mass% or more, 0.0005 mass% or more, and 0.001 mass% or more. In addition, the content of EGT can be, for example, 99.999 mass% or less with respect to the total amount of the composition, including 99.9 mass% or less, 99.5 mass% or less, 99 mass% or less, 98.5 mass% or less, and 98 mass% or less. In another embodiment, when prepared as a liquid formulation, the content of EGT is not limited, but can be, for example, 80 mass% or less with respect to the total amount of the composition, including 70 mass% or less, 60 mass% or less, 50 mass% or less, 40 mass% or less, 30 mass% or less, 20 mass% or less, 10 mass% or less, 5 mass% or less, and 1 mass% or less.

### [Use]

In a composition comprising EGT and SRH, reducing the content of the SRH can enhance the stability, and thus the present invention can be used to suppress coloring of the composition. An aspect of the present disclosure thus relates to the use, in a composition comprising L-ergothioneine and S-ribosylhomocysteine, of 15 parts by mass or less of S-ribosylhomocysteine with respect to 100 parts by mass of L-ergothioneine to suppress coloring of the composition. Another aspect relates to a method for imparting a coloring-suppressing effect to a composition comprising L-ergothioneine and S-ribosylhomocysteine, wherein the method comprises setting the content of S-ribosylhomocysteine in the composition to 15 parts by mass or less with respect to 100 parts by mass of L-ergothioneine.

In addition, as described above, it is known that EGT has various physiological activities including antioxidant ability. Also in a composition comprising EGT and SRH, when using the present invention, the stability can be enhanced so that the EGT does not impair but can exhibit the inherent physiological activities.

Also in a composition comprising EGT and SRH, when using the present invention, the composition can be suitably used for antioxidation, for improving brain function, for anti-aging, for eye diseases, for whitening, for absorbing ultraviolet rays, for inhibiting melanin production, for scavenging reactive oxygen species, for inhibiting elastase activity, for inhibiting wrinkle formation, for inhibiting skin sagging, for inhibiting formation of skin spots, for suppressing dark circles around eyes, for reducing skin damage by ultraviolet rays (inhibiting photoaging), for dry skin, for sensitive skin, for improving hair, for promoting autophagy, and the like, which are the inherent physiological activities of EGT.

The composition of the present invention can further appropriately contain an active component or an additive that can be used for foods or drinks, foods with functional claims, foods for specified health uses, quasi-drugs, pharmaceutical products, cosmetics, daily necessities, feeds, and the like, and can be appropriately formulated by a publicly known formulation method used for these items.

As cosmetics and daily necessities, for example, the composition of the present invention can be formulated into a skin lotion, an emulsion, a gel, a serum, a cream, a sunscreen cream, a pack, a mask, a foundation, a face powder, a bathing agent, a body lotion, a shampoo, a rinse, a hair treatment, a hair conditioner, a hairdressing, a hair tonic, a toothpowder, a mouthwash, and the like.

### [Formulation]

The composition of the present invention can be administered orally or parenterally (including externally) as, for example, a solid formulation such as a tablet, a capsule, a granule, or a powder; or as a liquid formulation such as a solution, a syrup, an injection, a cream, a lotion, a paste, an ointment, an emulsion (an oil-in-water emulsion, a water-in-oil emulsion, a multiple emulsion, a micro-emulsion, a PET-emulsion, a pickering emulsion), a gel (a hydrogel, an alcohol gel), or a suspension. For the solid formulation, an excipient, a lubricant, a binder, and a disintegrant can be used; and for the liquid formulation, a solvent, a solubilizing agent, an emulsifier, an emulsion stabilizer, a thickener, a humectant, a suspending agent, an isotonizing agent, a buffering agent, an analgesic agent, and the like can be used. If necessary, an additive such as an antiseptic, an antioxidant, a colorant, a sweetener, or a flavoring agent can also be used.

Examples of the excipient include sugar alcohols such as sorbitol, mannitol, and xylitol, saccharides such as glucose, white sugar, lactose, and fructose, crystalline cellulose, carmellose sodium, croscarmellose sodium, calcium hydrogen phosphate, wheat starch, rice starch, corn starch, potato starch, dextrin, β-cyclodextrin, light anhydrous silicic acid, titanium oxide, magnesium aluminate metasilicate, talc, kaolin, and olive oil.

Examples of the binder include cellulose derivatives such as methyl cellulose, ethyl cellulose, hydroxypropyl cellulose, and hydroxypropyl methyl cellulose, polyvinyl pyrrolidone, polyvinyl alcohol, acrylic acid-based polymers, gelatin, gum arabic, pullulan, pregelatinized starch, agar, tragacanth, sodium alginate, and propylene glycol alginate.

Examples of the disintegrant include starch, low-substituted hydroxypropyl cellulose, carboxymethyl cellulose calcium, croscarmellose sodium, hydroxypropyl starch, and partially pregelatinized starch.

Examples of the solvent include water, alcohol, propylene glycol, macrogol, sesame oil, and corn oil.

Examples of the lubricant include stearic acid, magnesium stearate, calcium stearate, polyoxyl stearate, cetanol, talc, hardened oil, sucrose fatty acid ester, dimethylpolysiloxane, beeswax, and white beeswax.

Examples of the solubilizing agent include polyethylene glycol, propylene glycol, mannitol, benzyl benzoate, ethanol, tris(hydroxymethyl)aminomethane, cholesterol, triethanolamine, sodium carbonate, and sodium citrate.

Examples of the suspending agent/emulsifier include surfactants such as stearylamine, triethanolamine, sodium lauryl sulfate, laurylaminopropionic acid, lecithin, benzalkonium chloride, benzethonium chloride, and glyceryl monostearate; hydrophilic polymers such as polyvinyl alcohol, polyvinylpyrrolidone, sodium carboxymethylcellulose, methylcellulose, hydroxymethylcellulose, hydroxyethylcellulose, and hydroxypropylcellulose; and waxes such as shellac wax, beeswax, carnauba wax, spermaceti wax, lanolin, liquid lanolin, reduced lanolin, hard lanolin, cyclic lanolin, lanolin wax, candelilla wax, Japan wax, montan wax, and rice wax.

Examples of the isotonizing agent include sodium chloride, glycerin, and D-mannitol.

Examples of the buffering agent include phosphate, acetate, carbonate, and citrate buffers.

Examples of the antiseptic include paraoxybenzoic acid esters, chlorobutanol, benzyl alcohol, phenethyl alcohol, dehydroacetic acid, and sorbic acid.

Examples of the antioxidant include sulfite and ascorbic acid.

When the composition of the present invention is formed into a solid formulation, a production method publicly known in the art can be used. Examples of the method include a method in which a product extruded, granulated, and molded by kneading a composition and passing the kneaded composition through a screen is pulverized and sized, and a method in which stirring granulation is performed by adding kneading water to the composition, followed by molding using a vertical granulator, and subsequently the granulated product is pulverized with a comil, and sieved. In addition, a method is included in which the formulation composition is compressed with a roller compactor, then pulverized with a roll granulator, and sieved. Furthermore, a method is included in which fluidized bed drying is performed after stirring granulation. Moreover, for example, in the case of producing by direct compression, a blended composition may be directly put into a tablet pressing machine and compressed.

### [Food and drink]

The composition of the present invention can also be used as a food and drink composition, or can be provided in a state of being comprised in foods or functional foods. Examples of such foods or functional foods include rice; various noodles including buckwheat noodles, Japanese wheat noodles, harusame noodles, Chinese noodles, instant noodles, and cup noodles; drinks such as soft drink, carbonated drink, nutritional drink, fruit drink, lactic acid drink, and sport drink; curry roux, stews, and various soups; frozen confectionery such as ice cream, ice sherbet, and shaved ice; confectionery such as Japanese candies, cookies, candies, gums, chocolates, tablet confectionery, snack confectionery, biscuits, jellies, jams, creams, and other baked confectionery; fish and livestock processed foods such as kamaboko, hanpen, hams, and sausages; dairy products such as processed milk and fermented milk; oils and fats, and oil-and-fat processed foods such as salad oil, tempura oil, margarine, mayonnaise, shortening, whipped cream, and dressing; condiments such as sauce, dressing, miso, soy sauce, and gravy; soups, salads, side dishes, furikake, and pickled vegetables; and various other forms of health and nutrition supplements, foods with functional claims, and foods for specified health uses.

In addition, supplements (such as powders, granules, soft capsules, hard capsules, tablets, chewable tablets, fast dissolving tablets, syrups, and liquid formulations) comprising the composition of the present invention may be prepared.

The composition of the present invention can also be comprised in feeds for animals such as pets.

If necessary, additives are added to foods or drinks. Examples of such additives include glucose, fructose, sucrose, maltose, sorbitol, trehalose, stevioside, rubusoside, corn syrup, lactose, mannitol, dextrin, citric acid, sodium citrate, tartaric acid, malic acid, succinic acid, lactic acid, L-ascorbic acid, tocopherol, sodium erythorbate, glycerin, propylene glycol, glycerin fatty acid esters, polyglycerin fatty acid esters, sucrose fatty acid esters, sorbitan fatty acid esters, gum arabic, carrageenan, casein, gelatin, pectin, agar, vitamins B, nicotinic acid amide, calcium pantothenate, amino acids, calcium salts, surfactants, dyes, flavors, and preservatives.

The composition of the present invention can be used for foods or drinks for which indication of improvement, prevention, progress or the like of various symptoms or conditions is permitted. Here, in the present invention, foods or drinks for which indication of improvement, prevention, progress, or the like of symptoms or conditions is permitted are foods and drinks having an efficacy permitted/designated by the government or a public organization, and are, for example, foods with functional claims, health functional foods such as foods for specified health uses, and special-use foods. Note that the names and regulations of such foods and drinks vary depending on the situation, the times, and the system in each country, but those that are essentially the same are included in the present invention.

In the present invention, the blending amount of the composition of the present invention is not particularly limited, and is appropriately set depending on the purpose of application (target disease, type of symptom, etc.), applied target site, sex and age of the applied subject, product forms such as foods and drinks, foods with functional claims, foods for specified health uses, quasi-drugs, pharmaceuticals, cosmetics, daily necessities, or feeds, the method and number of administration or ingestion of them, and preference.

When the composition of the present invention is used, it is possible to set an effective amount that can exhibit the inherent physiological activities of EGT as an ingesting amount (applied amount) per day. For example, when ingested by (applied to) a healthy adult, the ingesting amount (applied amount) of EGT per day can be used at, for example, 0.005 to 4,000 mg, preferably 0.1 to 3,000 mg, more preferably 0.5 to 2,000 mg, still more preferably 1 to 1,000 mg, particularly preferably 2 to 500 mg, most preferably 3 to 300 mg.

When the composition of the present invention is used as foods or drinks, the foods or drinks are not limited, but preferably functional foods from the viewpoint of being able to display the inherent physiological activities of EGT. Among the functional foods, foods with functional claims, nutritive functional foods, nutrition supplements, and foods for specified health uses are exemplified. Among them, foods with functional claims are preferable from the viewpoint of being able to clearly display the use.

### [Applied subject]

A subject to which the composition of the present invention is applied is not particularly limited as long as the subject is in the age group requiring the inherent physiological activities of EGT, but the subject may be around 30 years old or older, which is in an age group that is likely to receive stress from living environment such as work, may be a middle-aged person (around 45 years old or older and under 55 years old) who is in an age group that is likely to feel a change in the body due to a change in hormone balance, a change in living environment, or the like, or may be an aged person (55 years old or older).

### [pH]

The pH of the composition of the present invention is appropriately set depending on the type and content of other blending components, the formulation form, the method of use, and the like, and is not limited as long as the pH is within a pharmaceutically or physiologically acceptable range, but can be, for example, pH 2 to 10. From the viewpoint of stably exhibiting the effect of the present invention, the pH of the composition of the present invention can be, for example, pH 2 to 10, pH 2 to 9, pH 2 to 8, pH 2 to 7, pH 3 to 10, pH 3 to 9, pH 3 to 8, pH 3 to 7, pH 4 to 10, pH 4 to 9, pH 4 to 8, pH 4 to 7, pH 5 to 10, pH 5 to 9, pH 5 to 8, pH 5 to 7, pH 6 to 10, pH 6 to 9, pH 6 to 8, or pH 6 to 7.

### [Method for producing composition comprising EGT and SRH and having suppressed coloring]

In the present invention, the method for producing a composition comprising EGT and SRH and having suppressed coloring includes the step of reducing the content of SRH.

As the step of reducing the content of SRH, any publicly known means can be utilized as long as the SRH content is reduced. For example, when EGT and SRH are mixed to prepare a composition, a step of reducing the content ratio of SRH to EGT can be adopted. As described above, the mode in which SRH coexists in a composition comprising EGT is not particularly limited, and may be, for example, a mode in which SRH exists in impurities in an EGT extraction method, or a mode in which SRH exists as by-products in a synthesis method, a fermentation method, or the like.

As a method for producing EGT by a fermentation method, a method using E. coli or the like is publicly known from WO 2019/163767 A and the like.

Examples of the bacteria belonging to Enterobacteriaceae having EGT production ability include, but are not limited to, bacteria belonging to the genera Escherichia, Enterobacter, Pantoea, Klebsiella, and Salmonella. Particularly preferable examples of the enterobacteria include enterobacteria of Escherichia such as Escherichia coli and Pantoea such as Pantoea ananatis, and the like.

The culture of bacteria belonging to Enterobacteriaceae can be performed by a standard method. Specifically, an LB medium, a 2 × YT medium, an NZY medium, an M9 medium, an SOC medium, a YPD medium, or the like can be used. The medium described above can be used to produce EGT and SRH, but the medium to be used is not limited thereto. The produced EGT and SRH may be accumulated in the bacterial cells, or may be secreted and accumulated outside the cells (in the culture solution).

The collection of EGT and SRH that are present in the bacterial cells or liberated from the bacterial cells can be performed by a publicly known purification method or the like. For example, an extract of EGT and SRH can be obtained by subjecting the culture to solid-liquid separation such as centrifugation or filtration, and an extract thereof in the bacterial cell can be obtained by solvent extraction, hot water extraction, crushing treatment, or the like. From the extract of EGT and SRH or the culture supernatant, the content of SRH can be reduced by methods such as solvent extraction, separation by the solubility difference, chromatography such as chromatography using an adsorbent such as silica gel or alumina, ion exchange chromatography, hydrophobic chromatography, gel filtration chromatography, thiopropyl-sepharose 6B chromatography, or reversed phase chromatography, methods such as crystallization, activated carbon treatment, membrane treatment, treatment using an adsorbent or an ion exchange resin, and methods combining these methods.

Among these methods, from the viewpoint of efficiently reducing SRH in the composition comprising EGT and SRH, for example, methods such as chromatography using an adsorbent, ion exchange chromatography, crystallization, activated carbon treatment, treatment using an adsorbent or an ion exchange resin, and methods combining these methods are preferable.

In addition to the above, without limitation, in the composition comprising EGT and SRH, a means for decomposing the SRH may be used from the viewpoint of efficiently reducing SRH. Examples of the method for decomposing SRH include methods such as basifying, heating, and concentrating a solution of the composition, which may be appropriately combined with the purification method and the like described above.

In the various means for reducing the content of SRH, the preferred content ratio of SRH to EGT is as described above.

The production method may further include a step of adjusting the pH. The numerical range of the optimal pH is as described above.

### [Method for imparting coloring-suppressing effect to composition comprising EGT and SRH]

In the present invention, it is also possible to provide a method for imparting a coloring-suppressing effect to a composition comprising EGT and SRH by setting the content of the SRH to 15 parts by mass or less with respect to 100 parts by mass of the EGT. The preferred content ratio of SRH to EGT is as described above.

### EXAMPLES

Next, a specific description is made of the present invention with reference to Examples and Test Examples, but the present invention is not limited to the following Examples and Test Examples. When there is no particular description, the experiment was performed using the method described in the standard protocol collection related to molecular biology and applied microbiology, or a modified or altered method.

### (Test Example 1. Stability evaluation in composition comprising EGT and SRH)

For compositions comprising L-ergothioneine (EGT) and S-ribosylhomocysteine (SRH), the storage stability at 30°C was examined as follows. As a test model for confirming the influence of the content of SRH, a method for adding SRH to a composition comprising EGT is adopted, but EGT and SRH may be endogenously comprised as, for example, impurities in an extraction method, or by-products in a synthesis method, a fermentation method, or the like.

The SRH was synthesized according to the method described in CARBOHYDRATE RESEARCH, 2014, Vol. 394, page 32, and an SRH aqueous solution undergoing desalting by electrodialysis (comprising 0.108 w/w% SRH, quantified by NMR) was prepared.

Samples having an SRH content of 20 parts by mass, 15 parts by mass, 10 parts by mass, 5 parts by mass, 1 part by mass, 0.5 parts by mass, 0.1 parts by mass, or 0.01 parts by mass with respect to 100 parts by mass of EGT were prepared as follows.
(1) Dissolved was 500 mg of EGT (manufactured by Tetrahedron) in 50 mL. From this were sampled 5 mL × 8, in each of which was comprised an SRH 0.108 w/w% aqueous solution (9.3 mL, 6.98 mL, 4.63 mL, 2.33 mL, 0.463 mL, 0.232 mL, 46.3 µL, or 4.63 µL), to prepare samples comprising 20 parts by mass, 15 parts by mass, 10 parts by mass, 5 parts by mass, 1 part by mass, 0.5 parts by mass, 0.1 parts by mass, and 0.01 parts by mass of SRH with respect to 100 parts by mass of EGT.
(2) Each sample was divided into five equal parts, followed by freeze drying.
(3) Immediately after the freeze drying, each content was dissolved in 1 mL of water. Then, 200 µL of the solution was collected in a 96 well microplate, and the absorbance at 230 to 600 nm was measured using MULTISKAN GO (manufactured by Thermo SCIENTIFIC).
(4) The sample was stored at 30°C, and after 3 days, 24 days, and 6 months, each content was dissolved in 1 mL of water for absorbance measurement at 230 to 600 nm. Table 1 summarizes the SRH content with respect to EGT and change in absorbance using the absorbance data at 400 nm in (3) and (4) above.

**[Table 1]**

| SRH content with respect to 100 parts by mass of EGT | Day 0 | Day 3 | Day 24 | Month 6 |
|---|---|---|---|---|
| 20 parts by mass | 0.0118 | 0.0338 | 0.1765 | 0.7623 |
| 15 parts by mass | 0.0097 | 0.0295 | 0.1394 | 0.4188 |
| 10 parts by mass | 0.0067 | 0.0166 | 0.0763 | 0.2938 |
| 5 parts by mass | 0.0051 | 0.0178 | 0.0506 | 0.1931 |
| 1 part by mass | 0.0046 | 0.0075 | 0.0313 | 0.0832 |
| 0.5 parts by mass | 0.0033 | 0.0079 | 0.0203 | 0.0507 |
| 0.1 parts by mass | 0.0033 | 0.0033 | 0.0081 | 0.0167 |
| 0.01 parts by mass | 0.0034 | 0.0027 | 0.0049 | 0.0091 |

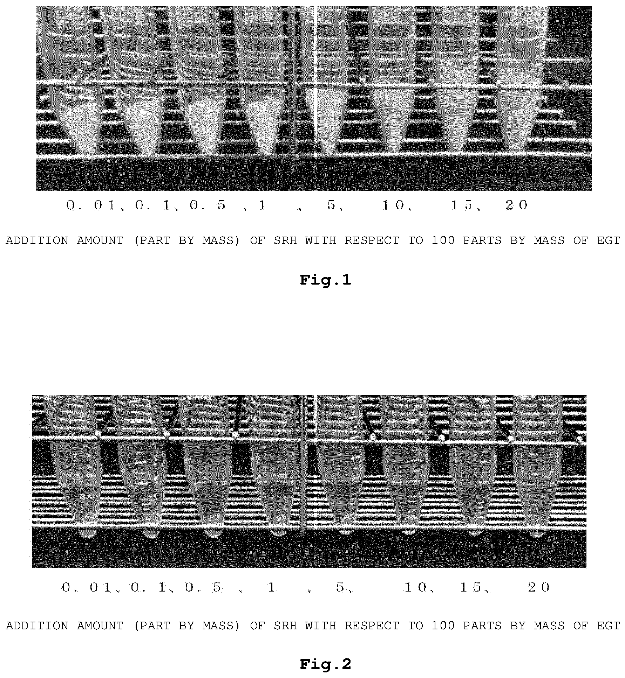

In addition, a photograph of the sample stored for 6 months before dissolution in water is shown in Fig. 1, and a photograph of the sample after dissolution in water is shown in Fig. 2.

As shown in Table 1 and Figs. 1 to 2, it was confirmed that among the samples stored at 30°C for 6 months, the lower the content of SRH, the more effective the coloring-suppressing effect of the composition. Without limitation, in the composition in which EGT and SRH coexist, when the content of SRH was 15 parts by mass or less with respect to 100 parts by mass of EGT, the absorbance immediately after storage was less than 0.01, and coloring could be remarkably suppressed even in long-term storage at 30°C.

### (Test Example 2. EGT crystallization from composition comprising EGT and SRH)

To 50 mg of EGT (manufactured by Tetrahedron) was added 2.31 mL of a 0.108 w/w% SRH aqueous solution (2.5 mg, 5 parts by mass SRH content with respect to 100 parts by mass of EGT). After concentration, the mixture was dissolved in 200 µL of water at 60°C, followed by addition of 2 mL of ethanol to precipitate crystals. The precipitated crystals were filtered at room temperature. The yield of the obtained EGT was 34 mg, and the HPLC quantitative value was 98.5% (the quantitative value was given by absolute quantification using an EGT manufactured by Tetrahedron as a standard). Similarly, the results of crystallization performed in case of 10 parts by mass of SRH with respect to 100 parts by mass of EGT and the results of crystallization performed in case of 20 parts by mass of SRH are shown in Table 2.

### (HPLC analysis condition)

Quantification of EGT was performed under the following HPLC conditions.
Column: YMC-Pack ODS-AQ S-5 µM, 12 nm, 250 × 4.6 mm I.D.
Flow rate: 0.5 mL/min, Temperature: 30°C
Detector: PDA (257 nm)
Eluent: 0.1% aqueous formic acid solution
Retention time: around 8.9 minutes

**[Table 2]**

| SRH content with respect to 100 parts by mass of EGT | EGT yield | HPLC quantitative value |
|---|---|---|
| 20 parts by mass | 30 mg | 83.1% |
| 10 parts by mass | 35 mg | 97.8% |
| 5 parts by mass | 34 mg | 98.5% |

As shown in Table 2, in the composition in which EGT and SRH coexist, when the content of SRH is 20 parts by mass with respect to 100 parts by mass of EGT, a new problem was found in that the yield when performing crystallization of EGT and the HPLC quantitative value decrease. On the other hand, in the composition in which EGT and SRH coexist, when the content of SRH is 15 parts by mass or less (10 parts by mass and 5 parts by mass) with respect to 100 parts by mass of EGT, it was confirmed that the yield when performing crystallization of EGT and the HPLC quantitative value were remarkably improved.

### [Reference Example]

In an SRH 0.108 w/w% aqueous solution (1 mL) was dissolved 7.1 mg of EGT (manufactured by Tetrahedron) to prepare a sample having 15 parts by mass of SRH with respect to 100 parts by mass of EGT.

For HPLC analysis, 100 µL of the sample was diluted 10 times with water. The remaining 900 µL was concentrated under reduced pressure using an evaporator at 30°C. After the mixture was left to stand at room temperature for 4 days, the mixture was dissolved in 0.9 mL of water. For HPLC analysis, 100 µL of the solution was diluted 10 times with water. The result of comparing the HPLC area with that before concentration is shown in Table 3.

**[Table 3]**

| Retention time | Before concentration | 4 days after concentration |
|---|---|---|
| SRH Around 6.6 minutes | 156690 | 108189 |
| SRH dehydrated dimer Around 7.5 minutes | 6733 | 41309 |
| EGT Around 8.7 minutes | 13959268 | 14131254 |

As shown in Table 3, the HPLC area of the peak around 7.5 minutes increased with the decrease in the HPLC area of SRH. In addition, this peak was presumed to be an SRH dehydrated dimer through high-resolution mass spectrometry. Analysis conditions for HPLC and analysis conditions for high resolution mass spectrometry (LC-MS) are as follows.

### (HPLC analysis condition)

HPLC analysis was performed under the following conditions.
Column: YMC-Pack ODS-AQ S-5 µM, 12 nm, 250 × 4.6 mm I.D.
Flow rate: 0.5 mL/min, Temperature: 30°C
Detector: PDA (210 nm)
Eluent: 0.1% aqueous formic acid solution
Injection amount: 5 µL
Retention time: SRH: around 6.6 minutes; SRH dehydrated dimer: around 7.5 minutes; EGT: around 8.7 minutes.

### (High-resolution mass spectrometry)

The results of high-resolution mass spectrometry for the SRH dehydrated dimer are shown below.
[M+H]⁺ calculated value for C₁₈H₃₃N₂O₁₁S₂ 517.1527; measured value 517.1522
High resolution mass spectrometry (LC-MS) analysis condition) Apparatus: Agilent Technologies 6224 TOF LC/MS (manufactured by Agilent Technologies)
Column: YMC-Pack ODS-AQ S-5 µM, 12 nm, 250 × 4.6 mm I.D.
Flow rate: 0.5 mL/min, Temperature: 30°C
Eluent: 0.1% aqueous formic acid solution
Ionization conditions: ESI; Capillary voltage: 3500 V; Fragmentor voltage: 100 V
Retention time: SRH: around 6.5 minutes; SRH dehydrated dimer: around 7.3 minutes; EGT: around 8.5 minutes.

## Claims

1. A composition comprising L-ergothioneine and S-ribosylhomocysteine, wherein the content of S-ribosylhomocysteine is 15 parts by mass or less with respect to 100 parts by mass of the L-ergothioneine.

2. The composition according to claim 1, wherein the content of S-ribosylhomocysteine is 5 parts by mass or less with respect to 100 parts by mass of the L-ergothioneine.

3. The composition according to claim 1 or claim 2, wherein the content of S-ribosylhomocysteine is 0.001 parts by mass or more with respect to 100 parts by mass of L-ergothioneine.

4. Use, in a composition comprising L-ergothioneine and S-ribosylhomocysteine, of 15 parts by mass or less of S-ribosylhomocysteine with respect to 100 parts by mass of L-ergothioneine to suppress coloring of the composition.

5. A method for imparting a coloring-suppressing effect to a composition comprising L-ergothioneine and S-ribosylhomocysteine, wherein the method comprises setting the content of S-ribosylhomocysteine in the composition to 15 parts by mass or less with respect to 100 parts by mass of L-ergothioneine.

## Patentansprüche

1. Eine Zusammensetzung, die L-Ergothionein und S-Ribosylhomocystein enthält, wobei der Gehalt an S-Ribosylhomocystein 15 Masseteile oder weniger bezogen auf 100 Masseteile des L-Ergothioneins beträgt.

2. Die Zusammensetzung nach Anspruch 1, wobei der Gehalt an S-Ribosylhomocystein 5 Masseteile oder weniger, bezogen auf 100 Masseteile des L-Ergothioneins, beträgt.

3. Die Zusammensetzung nach Anspruch 1 oder Anspruch 2, wobei der Gehalt an S-Ribosylhomocystein 0,001 Masseteile oder mehr bezogen auf 100 Masseteile L-Ergothionein beträgt.

4. Verwendung in einer Zusammensetzung, die L-Ergothionein und S-Ribosylhomocystein umfasst, von 15 Massenanteilen oder weniger S-Ribosylhomocystein bezogen auf 100 Massenanteile L-Ergothionein um die Verfärbung der Zusammensetzung zu unterdrücken.

5. Verfahren zum Verleihen einer färbungsunterdrückenden Wirkung an eine Zusammensetzung, die L-Ergothionein und S-Ribosylhomocystein umfasst, wobei das Verfahren das Einstellen des Gehalts an S-Ribosylhomocystein in der Zusammensetzung auf 15 Masseteile oder weniger bezogen auf 100 Masseteile L-Ergothionein umfasst.

## Revendications

1. Composition comprenant de la L-ergothionéine et de la S-ribosylhomocystéine, dans laquelle la teneur en S-ribosylhomocystéine est de 15 parties en masse ou moins pour 100 parties en masse de L-ergothionéine.

2. Composition selon la revendication 1, dans laquelle la teneur en S-ribosylhomocystéine est de 5 parties en masse ou moins pour 100 parties en masse de L-ergothionéine.

3. Composition selon la revendication 1 ou la revendication 2, dans laquelle la teneur en S-ribosylhomocystéine est de 0,001 partie en masse ou plus pour 100 parties en masse de L-ergothionéine.

4. Utilisation, dans une composition comprenant de la L-ergothionéine et de la S-ribosylhomocystéine, de 15 parties en masse ou moins de S-ribosylhomocystéine pour 100 parties en masse de L-ergothionéine pour supprimer la coloration de la composition.

5. Méthode pour donner un effet de suppression de coloration à une composition comprenant de la L-ergothionéine et de la S-ribosylhomocystéine, laquelle méthode comprend la fixation de la teneur en S-ribosylhomocystéine dans la composition à 15 parties en masse ou moins pour 100 parties en masse de L-ergothionéine.
